Europäisches Patentamt

European Patent Office

Office européen des brevets

(19)

(11) Veröffentlichungsnummer: **0 237 751**
**B1**

(12) **EUROPÄISCHE PATENTSCHRIFT**

(45) Veröffentlichungstag der Patentschrift: **29.08.90**

(21) Anmeldenummer: **87101573.1**

(22) Anmeldetag: **05.02.87**

(51) Int. Cl.⁵: **A 61 F 2/34**

(54) Endoprothese für eine Hüftgelenkspfanne.

(30) Priorität: **18.02.86 CH 651/86**

(43) Veröffentlichungstag der Anmeldung:
**23.09.87 Patentblatt 87/39**

(45) Bekanntmachung des Hinweises auf die
Patenterteilung:
**29.08.90 Patentblatt 90/35**

(84) Benannte Vertragsstaaten:
**AT DE FR GB IT**

(56) Entgegenhaltungen:
**EP-A-0 028 546**
**EP-A-0 144 588**
**CH-A- 649 913**
**DE-A-2 834 298**

(73) Patentinhaber: **GEBRÜDER SULZER
AKTIENGESELLSCHAFT
Zürcherstrasse 9
CH-8401 Winterthur (CH)**

(72) Erfinder: **Stuehmer, Karl-Gerhart, Dr.-med.
St. Elisabethen-Krankenhaus
D-7980 Ravensburg (DE)**

(74) Vertreter: **Sparing Röhl Henseler Patentanwälte
European Patent Attorneys
Rethelstrasse 123
D-4000 Düsseldorf 1 (DE)**

## Beschreibung

Die Erfindung betrifft eine Endoprothese für eine Hüftgelenkspfanne zur zementfreien Verankerung im menschlichen Körper, bestehend aus einer halbkugelförmigen Aussenschale, deren äussere Oberfläche mit einem Schraubengewinde konstanter Steigung versehen ist, dessen Gewindegänge durch in Meridianrichtung verlaufende Einschnitte unterteilt sind, und aus einem in dieser Aussenschale, die aus Reintitan oder einer Titanlegierung gefertigt ist, fixierten Pfannenkörper.

Eine Prothese der vorstehend genannten Art ist aus der EP—A—0 144 588 bekannt. Bei derartigen Prothesen, bei denen die Pfannenkörper wegen der guten Gleiteigenschaften vorwiegend aus Kunststoff, beispielsweise aus Polyäthylen, und die Aussenschalen aus Festigkeitsgründen aus körperverträglichen Metall, beispielsweise aus Reintiten, gefertigt sind, besteht allgemein das Problem, dass die Elastizität der Aussenschale sehr stark von derjenigen des Beckenknochens abweicht. Die der Erfindung zugrundeliegende Aufgabe besteht daher zum einen darin, der Aussenschale der Prothese und vor allem ihren in den Rechenknochen einschneidenden Gewindegängen eine möglichst grosse Elastizität zu geben, ohne ihre Festigkeitseigenschaften unzulässig zu beeinträchtigen und ohne eine unerwünschte plastische, d.h. bleibende, Verformung zu "induzieren". Zum anderen übt der Gelenkkopf, der im allgemeinen ebenfalls Teil einer implantierten Prothese ist. Kippmomente auf die Pfannenprothese aus, die möglichst optimal in den Beckenknochen weiterzuleiten sind.

Diese doppelte Aufgabe wird erfindungsgemäss dadurch gelöst, dass für die Fussbreiten und die Spitzenbreiten der Gewindegänge bei Absolutwerten für die Länge der Gewindegänge 4 mm≤L≤12 mm folgende Beziehungen gelten: 0,025 L≤A≤0,05 L und 0,15 L≤A≤0,2 L und dass ferner im Polbereich der Aussenschale kreisringförmige Schneidnuten angeordnet sind.

Wie aufgrund von Versuchen ermittelt worden ist, bieten—für Aussenschalen aus den eingangs genannten Werkstoffen—die beanspruchten Relationen für die Abmessungen der Gewindegänge des Schraubengewindes Gewähr für eine optimale Erfüllung der genannten, mindestens zum Teil an sich wiedersprüchlichen Forderungen; durch die Schneidkanten im Polbereich der Aussenschale wird eine gute Uebertragung der Kippmomente auf den Knochen gewährleistet, und damit allgemein die Haftung der Prothese im Beckenknochen verbessert.

Weitere Vorteile der erfindungsgemässen Konstruktion sind eine relativ grosse Auflage- und Abstützfläche der Prothese in Richtung ihrer Achse.

Im folgenden wird die Erfindung anhand eines Ausführungsbeispiels im Zusammenhang mit der Zeichnung näher erläutert.

Fig. 1 zeigt die neue Endoprothese in einer Seitenansicht, die teilweise in einem Meridianschnitt dargestellt ist;

Fig. 2 ist—ebenfalls in einem Meridianschnitt—eine vergrösserte Darstellung des äquatorialen Bereichs der Konstruktion nach Fig. 1.

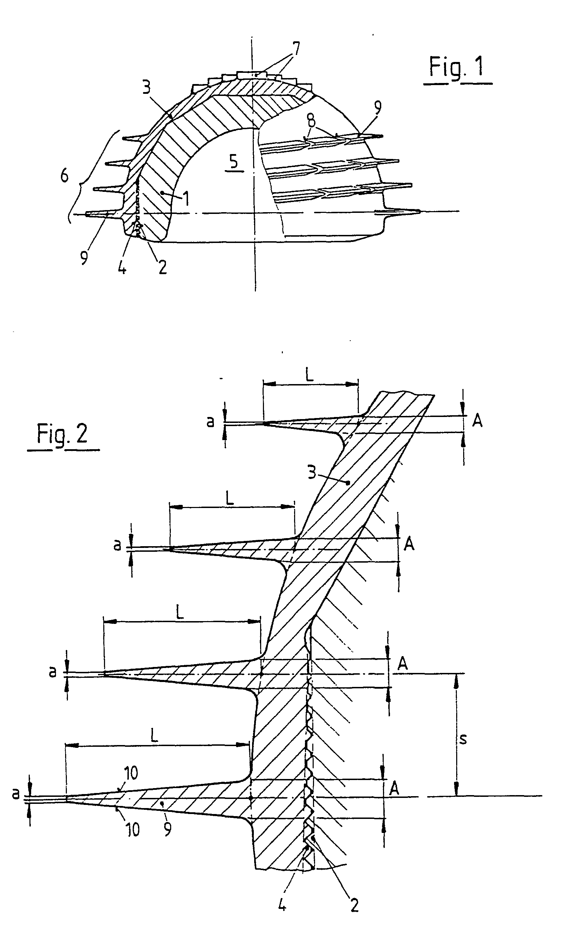

Die Endoprothese nach Fig. 1 hat einen Pfannenkörper 1, dessen äussere Begrenzung im Querschnitt eine Vieleck bildet; die äquatorialen Seiten dieses Vieleckes sind mit einem Gewinde 2 versehen; über das Gewinde 2 und ein entsprechendes Gegengewinde 4 wird der Pfannenkörper 1 im an seiner Aussehbegrenzung angepassten Hohlraum einer metallischen Aussenschale 3 fixiert.

Von der Seite des Aequators her ist in den Pfannenkörper 1 die eigentliche Pfannenschale 5 in Form einer hohlen Halbkugel eingearbeitet, die den Gelenkkopf einer nicht gezeigten Femurkopfprothese aufnimmt.

Die äussere, ebenfalls halbkugelförmige Oberfläche, mit der die Aussenschale 3 im Beckenknochen verankert wird, trägt auf ihrem Bereich "äquatornaher und mittlerer Breiten" in bekannter Weise ein mehrgängiges Schraubengewinde 6 mit konstanter Steigung s (Fig. 2). Darüberhinaus ist diese Oberfläche im Polbereich mit einer zusätzlichen Struktur versehen, die aus hervorstehenden, als Schneidkanten ausgebildeten Kreisringen 7 besteht. Beim Einschrauben der Prothese in den Beckenknochen schneiden sich diese Ringe 7 in das Knochengewebe eine und ergeben so eine zusätzliche Fixierung der Prothese und ein zusätzliches Widerlager für die erwähnten, von Femurkopf ausgeübten Kippmomente.

Die Länge L der Gewindegänge 9 des Schraubengewandes 6, die in Umfangsrichtung von jeweils in Meridianrichtung der Halbkugel verlaufenden Einschnitten 8 unterteilt sind, nimmt beim Fortschreiten vom Aequator zum Pol kontinuierlich ab. Die Länge L ist dabei, in der Mittelebene jedes Gewindeganges 9 gemessen, der Abstand zwischen der Halbkugeloberfläche und der Spitze des Gewandeganges 9. Sie bildet die Basis für die beanspruchten Beziehungen und beträgt in Absolutwerten bei Aussenschalen aus Titan oder Titanlegierungen 4—12 mm.

Die Grösse A, d.h. die Dicke eines Gewindeganges 9 an seinem Fusspunkt, ergibt sich aus den—an sich durch einen hohlkehlenartigen Radius abgerundeten—Ecken, die die Schnittpunkte der verlängerten Flanken 10 eines Gewindeganges 9 mit der Halbkugeloberfläche der Aussenschale 3 bilden, während die Dicke der einzelnen Gewindegänge 9 an ihrer Spitze mit a bezeichnet ist.

Zwischen den Grössen A, a und L bestehen die beanspruchten Beziehungen, wobei die Länge L die Bezugsgrösse ist, nach der die beiden anderen Abmessungen ausgerichtet werden.

Bei Einhaltung dieser Beziehungen ergibt sich ein optimales Verhältnis zwischen der Elastizität der Gewindegänge 9 einerseits und der Festigkeit sowie dem Widerstand gegen bleibende Verförmungen auf der anderen Seite.

Die Gewindegänge 9 werden bei der Fabrikation der Aussenschale 3 beispielsweise durch numerisch gesteuertes Gewindeschneiden oder durch Fräsen aus dem Vollen eines Rohlings herausgearbeitet.

## Patentansprüche

1. Endoprothese für eine Hüftgelenkspfanne zur zementfreien Verankerung im menschlichen Körper, bestehend aus einer halbkugelförmigen Aussenschale (3), deren äussere Oberfläche mit einem Schraubengewinde (6) konstanter Steigung (5) versehen ist, dessen Gewindegänge (9) durch in Meridianrichtung verlaufende Einschnitte (18) unterteilt sind, und aus einem in dieser Aussenschale (3), die aus Reintitan oder einer Titanlegierung gefertigt ist, fixierten Pfannenkörper (1), dadurch gekennzeichnet, dass für die Fussbreiten (A) und die Spitzenbreiten (a) der Gewindegänge (9) bei Absolutwerten für die Länge (L) der Gewindegänge (9) 4 mm≤L≤12 mm folgende Beziehungen gelten:

0,025 L≤a≤0,05 L und 0,15 L≤A≤0,2 L, und dass ferner im Polbereich der Aussenschale (3) kreisringförmige Schneidnuten (7) angeordnet sind.

2. Endoprothese nach Anspruch 1, dadurch gekennzeichnet, dass die Stiegung (s) des Gewindes 6,5 mm beträgt.

## Revendications

1. Endoprothèse pour une cavité cotyloïde, destinée à être ancrée sans ciment dans le corps humain, constituée d'une cogue extérieure (3) semi-sphérique dont la surface extérieure est pourvue d'un filetage (6), de pas (5) constant, dont les filets (9) sont subdivisés par des entailles (18) s'étendant dans la direction du méridien, ainsi que d'un corps de cavité (1), fixé dans cette coque extérieure (3) qui est en titane pur ou dans un alliage de titane, caractérisé en ce que pour les largeurs de pied (A) et les largeurs de sommet (a) des filets (9), la longueur (L) des filets (9) ayant des valeurs absolues telles que 4 mm≤L≤12 mm, on a les relations suivantes:

0,025 L≤a≤0,05 L et 0,15 L≤A≤0,2 L et en ce qu'en outre, des rainures de coupe (7), en forme d'anneau circulaire, sont placées dans la région du pôle de la coque extérieure (3).

2. Endoprothèse selon la revendication 1, caractérisée en ce que le pas (s) du filetage est de 6,5 mm.

## Claims

1. An endoprosthesis for an acetabulum for uncemented fixing in the human body, the endoprosthesis comprising: a hemispherical outer shell (3) whose outside surface has screwthreading (6) of a constant pitch (5), the screwthreading turns (9) being subdivided by incisions (18) in the meridian direction; and a socket (1) secured to the outer shell (3), the same being made of pure titanium or a titanium alloy, characterized in that for the root widths (A) and the crest widths (a) of the screwthread turns (9) with absolute values for the length (L) of the screwthread turns (9) of 4 mm≤L≤12 mm, the following relationships apply:

0,025 L≤a≤0,05 L and 0,15 L≤A≤0,2 L, and annular cutting grooves (7) are present in the polar zone of the outer shell (3).

2. An endoprosthesis according to claim 1, characterised in that the pitch (s) of the screwthread is 6.5 mm.

Fig. 1

Fig. 2